Europäisches Patentamt

European Patent Office

Office européen des brevets

(11) Publication number: **0 210 561**
**A2**

# (12) EUROPEAN PATENT APPLICATION

(21) Application number: 86109941.4

(22) Date of filing: 19.07.86

(51) Int. Cl.⁴: **A 61 K 7/035**
**C 08 J 9/28**

(30) Priority: 22.07.85 US 757252

(43) Date of publication of application:
04.02.87 Bulletin 87/6

(84) Designated Contracting States:
BE DE FR GB IT NL SE

(71) Applicant: DeSOTO, INC.
1700 South Mt. Prospect Road
Des Plaines Illinois 60018(US)

(72) Inventor: Brown, Wallace H.
1303 Gilbert
Downers grove, Ill.60515(US)

(72) Inventor: Vandeberg, John T.
415 West Oakwood drive
Barrington, Ill. 60010(US)

(72) Inventor: Dachniwskyj, Maryam L.
1320 Cambia Drive 7208
Schaumburg, Ill.60193(US)

(74) Representative: Reitzner, Bruno, Dr.
Patentanwälte Dipl.-Ing. R. Splanemann Dr. B. Reitzner
Tal 13
D-8000 München 2(DE)

(54) Dry powder cosmetic composition with sustained release of oily liquid.

(57) Vesiculated beads of cross-linked resin containing an average of at least 2 thin-walled foraminous cells to provide communication between the vesicles within the beads and the exterior of the beads to allow liquid to move into and out of the beads have an oily liquid incorporated therein after they are formed. The oily liquid may be a long chain ester, such as isopropyl myristate, and the beads containing the emollient are incorporated into dry powder cosmetic compositions, preferably together with talc or clay.

EP 0 210 561 A2

-1-

# DRY POWDER COSMETIC COMPOSITION WITH
# SUSTAINED RELEASE OF OILY LIQUID
## DESCRIPTION

### Technical Field

This invention relates to dry powder cosmetic compositions characterized by the sustained release of an oily liquid, usually an emollient, to provide a silky and smooth feeling when applied to skin, and especially to normally dry skin. The invention particularly contemplates face powders, body powders, baby (diaper) powder, and protective hand powder.

### Background Art

Cosmetic compositions intended to apply an oily liquid or emollient are in common use, but these compositions are liquids or creams, and not dry powders. As a result, these prior compositions provide an oily appearance, whereas it is desired to give the skin a dry appearance at the same time that the skin is moisturized or protected by the emollient and made to feel smoother and silkier.

It is possible to incorporate oily liquids into face powders and body powders, but then the powder particles stick together, making the powder difficult to handle and hard to apply. Moreover, little oil can be picked up by the powder particles, and the desired smooth and silky feel is not achieved.

Another possibility is to encapsulate the emollient within some sort of a tiny capsule, but this has presented a very limited choice. While the emollient is in the capsule, it is ineffective. After the capsule has been ruptured, the full contents of the capsule become available in one spot, and the emollient sticks the powder particles together at that spot which causes the agglomeration

-2-

difficulties previously noted.

Description of Invention

In accordance with this invention, the oily liquid or emollient is incorporated into substantially dry preformed vesiculated beads of cross-linked resin, the beads containing an average of at least 2, more usually at least 5, thin-walled foraminous cells which allow liquid to move into and out of the beads. These cells preferably include at least some cell walls which are discontinuous on electron microscope examination to provide communication between the vesicles within the bead and the exterior of the bead and thus enable movement of liquid by capillary action, though this action may not require openings large enough to be seen. The very thin walls of the cells (about 0.2 micron) are concluded to be foraminous, for water contained therein is easily removed by simple exposure to air even though no fissure large enough for the electron microscope to pick up is found in many of the cells.

It is found that these vesiculated beads can be loaded with oily liquid after they have been formed, and the added oil largely remains within or adsorbed onto the beads to be available for use even when the beads are blended with other powder components. In this way, powder compositions can be formulated with beads containing up to about their own weight of oily liquids. The powders may be free flowing, but can be used by spreading even when the particles cling together.

When these dry powder compositions are applied to the skin in a thin layer, the oily liquid is slowly released. In this way, the oil is present in a manner which limits its tendency to stick the powder particles together, and the oil is constantly renewed on the skin to maintain its presence thereon over an extended period of time. As a result, the emollient or other desired action is obtained despite the powder form of the composition, and this action is maintained over an extended period of time.

This invention includes a process of incorporating oily liquids into hollow plastic beads in a manner permitting the liquid to leave the beads when they are spread on a surface. More particularly, it is found that the vesiculated beads can be loaded with oily liquid after bead formation is complete, and the loaded material largely remains within the beads when the beads are covered, as when they are stored in a closed container alone or together with other materials prior to use. When the loaded beads are later applied as a thin layer or dust (onto the skin or other surface), the oily liquid is slowly released.

In this way, the oily liquid can be loaded into the vesiculated beads by simply mixing the two together and holding them together in admixture with one another to allow the oily liquid to enter the cells of the beads. Agitation can be used, but is not usually necessary. While all the liquid can be taken up, this is not necessary since additional powder can be added to absorb or adsorb that which is not taken up by the vesiculated beads.

The oily liquid may be employed in combination with inert volatile organic solvent, such as an alcohol like ethyl alcohol or propyl alcohol, or a ketone like acetone or methyl ethyl ketone. When a solvent is used, all or a portion of the solvent can be removed after the solution of the oily liquid has moved into the beads by allowing the solvent to evaporate. In this way one can use

-4-

liquids which are too viscous for easy handling in the absence of solvent.

These vesiculated beads with the emollient or other oily liquid within them can be applied alone, but are preferably applied in combination with other powder components, such as talc, or a clay, such as kaolin. Kaolin clay provides adhesion and is typically used in face powders in an amount of from 4-5%. The composition may include colorants or other additions for special purpose, such as perfumes, medicaments of various type, or sun blocking agents.

While other components providing special benefit can be incorporated into the powder compositions of this invention, the prime objective of this invention is to provide a powder composition which can be easily applied to the skin and which will provide a relatively dry surface appearance at the same time that it effectively applies an oily liquid to confer a smooth and silky feel and to add oil to a skin which may be excessively dry. Water repellent oils are also contemplated, and provide a water repellent action from a dry powder.

While the free flowing powder characteristic is not an essential of this invention, it can be obtained in various ways. Most simply, one can mix the vesiculated beads containing emollient with a large proportion of talc or clay, and the larger the proportion of admixed talc or similar particle, the less the tendency of the oil to cause agglomeration. It is preferred to employ powder compositions in which at least about 50% of the composition is constituted by talc or clay.

Another way to minimize agglomeration is to include a small proportion of fumed silica, and this is effective in amounts of from 2% up to about 20%, preferably from 3% to 10%.

Still another way to minimize agglomeration is to minimize the proportion of emollient in the vesiculated beads. Thus, while one may include from about 3% up to about 65%, preferably from 10% to 50% of the final weight of the emollient-containing beads, the less emollient in the beads, the less the tendency of these beads to induce agglomeration.

All sorts of oily liquids may be used, as is well known in the art. These are usually emollients, illustrated by long chain esters, such as isopropyl myristate. Other emollients which may be used are illustrated by mineral oil. Still other oils which may be used are methyl-terminated polysiloxane oils, and these provide a water repellent skin protecting powder. Also, the oily liquid may provide a vehicle for introducing all sorts of active agents, such as medicaments or sun blocking agents, into a dry powder composition.

Among the silicone oils, good results have been obtained using dimethicone (a low volatility silicone from General Electric Company SF 96-200 may be used) and cyclomethicone (a high volatility silicone from Union Carbide Corp. 7158 may be used).

The vesiculated beads to be useful in this invention must be substantially unground, for aggressive grinding breaks up the beads and impairs their usefulness. The beads may vary in average size of from about 1 micron to about 30 microns, but beads having an average size of from 3 microns to 20 microns are preferred. These beads preferably average at least about 5 vesicles per bead.

The beads in this invention are substantially free from water, and this denotes a water content of less than about 0.2% by weight. The

-6-

dried beads can be provided by removing water from the water-wet paste of vesiculated beads which is provided when these beads are produced by polymerization in aqueous emulsion.

Water removal can be by simple exposure to air at room temperature, by passing heated air, typically at a temperature of 100°F. to 120°F. over the beads, by tumbling the wet beads in a rotating structure such as a pipe or centrifuge basket while air is passed through the beads, by dropping them through a tower with drying gas moves upwardly therethrough, by vacuum drying, by using an azeotroping solvent, or in any other desired manner.

It should be noted that the beads are usually produced by polymerization in aqueous emulsion so that they are provided in aqueous medium. When this aqueous medium is filtered or allowed to separate with the supernatant aqueous liquor decanted, then the vesiculated beads which are desired are provided in the form of an aqueous cake which includes the surfactants and colloidal material involved in their production. These tend to cause agglomeration when simple drying is used, and this feature of the beads can be utilized in the production of pressed cakes. In most instances, the agglomeration tendency will be avoided by washing the beads with water one or more times to remove these adhesive agents with the wash water. Solvent washing is also permitted, and the water can be azeotropically removed by heating the solvent. Some of the solvent can be allowed to remain to help load the beads with the volatile liquid.

The water-wet paste which is treated in the above manner is formed when excess water is drained from the aqueous suspension obtained when the beads are prepared in aqueous suspension, a surfactant and/or protective colloid being needed to maintain the suspension while the beads are produced. A typical paste is called a cake, and it contains about 65% water while appearing to be dry.

The beads in this invention can be pigmented or unpigmented, depending upon the cosmetic application which is intended. When pigmentation is desired, the pigment can be incorporated into the walls of the beads as they are formed, and titanium dioxide and iron oxides are typical pigments. One can also associate with the preformed beads a pigment which is incompatible with an azeotropic organic solvent selected to help remove the water by azeotroping it off. Thus, one can add a water-based pigment, such as lamp black, to the aqueous paste either before or after the addition of organic azeotropic solvent to be present while the water is azeotropically removed. This causes the added pigment to deposit upon and adhere to the surface of the beads.

The vesiculated beads are produced in an aqueous suspension which is drained to form a water-wet paste. The procedure for producing these beads involves polymerization in aqueous emulsion in the presence of surfactants and, preferably also, protective colloids. Protective colloids such as polyvinyl alcohol and hydroxyethyl cellulose are frequently used. These agents are adhesive, and it is desired to remove them in many instances, but when a pressed cake form of dry powder is desired, these agents can be permitted to remain, thus minimizing the use of extraneous adhesive agents to hold the particles together in the pressed cake.

The preferred vesiculated beads are

-8-

styrene-cross-linked unsaturated polyester resins. These are made into a vesiculated bead in conventional fashion, as illustrated by U.S. Pat. No. 3,879,314. Various other patents are of interest to the formation of vesiculated beads useful in this invention, particular attention being directed to U.S. patents Nos. 3,822,224, 3,923,704 and 3,933,579. This last-named patent describes the vesiculated beads which are preferred herein, namely, those having a ratio of granular diameter to mean vesicle diameter of at least 5:1, a vesicle volume of from 5% to 95% of the volume of the granule, and not more than about 60% pigment, by volume.

The vesiculated beads used herein have a highly cross-linked polymeric body which is preferably constituted by a carboxyl-functional unsaturated polyester resin cross-linked with an ethylenically unsaturated monomer copolymerizable therewith. The unsaturation in the polyester is preferably maleate unsaturation, these polyesters being themselves well known and illustrated hereinafter. It is preferred that the polyester have an acid value of 10 to 45 mgm KOH per gm.

During the production process, water migrates into the polymeric beads to swell them, and the polymer in the bead walls polymerizes at the same time. As a result, it is normal to have some of the cell walls fracture, and the extent of fracturing can be controlled by controlling the polymerization process. The more polymerization can be delayed, the greater the number of cell walls which are either not present or disrupted. In this way, the proportion of volatile liquid which can be held by the vesiculated beads can be varied.

The unsaturated monomers used for cross-linking are also well known and are water insoluble monomers typically illustrated by styrene or vinyl toluene. The polyesters and monomers which may be used are more fully discussed in U.S. Pat. No. 3,879,314 which shows the production of vesiculated beads using a water-soluble polyamine containing at least three amine groups per molecule and having a dissociation constant in water (pKa value) of 8.5-10.5, typically illustrated by diethylene triamine. The polyamine is used in a concentration providing 0.3 to 1.4 amine groups per polyester carboxyl group. It is preferred to have from 35% to 45% of the unsaturated polyester cross-linked with from 55% to 65% of styrene.

Suitable pigmented vesiculated beads in accordance with this invention are illustrated in U.S. Patent No. 3,879,314 issued April 22, 1975, see particularly Example II. By proceeding in accordance with said Example II and using a polyester of 18% phthalic anhydride, 37% maleic anhydride and 45% propylene glycol dissolved in styrene to form a solution containing 41.8% of the polyester, vesiculated beads pigmented with titanium dioxide, anatase, to contain about 43.2% pigment are provided. These beads have an average size of about 9 microns and contain an average of more than 10 cells per bead, and are the beads used in the Examples of this application.

The invention is illustrated in the examples which follow. All parts and proportions herein are by weight unless otherwise specified.

Example 1

An aqueous slurry of vesiculated beads has the bulk of its water content removed by decantation to provide an aqueous paste (termed a beadcake) containing 35 parts of vesicular beads, 64.5 parts water, 0.18 parts of a 75% solution of sodium dioctyl sulfosuccinate (the American Cyanamid surfactant, Aerosol OT may be used), 0.28 parts polyvinyl alcohol and 0.04 parts hydroxy ethyl cellulose. These surfactants and colloids are typical residues of bead production and might cause agglomeration if the water were removed by simple drying. The vesiculated beads are composed of 56.0% resin and 44.0% titanium dioxide. The bead resin is an unsaturated polyester containing propylene glycol/maleic anhydride/phthalic anhydride in proportions of 3.72/2.06/4.22, and this polyester is dissolved in styrene to provide a 58/42 ratio of styrene to polymers. To form the beads, the mixture of polyester and styrene is dispersed in water with the aid of surfactants and protective colloids in the proportions noted and copolymerized in the presence of a quaternary ammonium salt to cause vesicles to form as described in U.S. Pat. No. 3,879,314. The resulting vesiculated beads are in water slurry, and this is drained to provide the beadcake starting material.

The beadcake is then dispersed in 50% of its volume of deionized water with agitation and allowed to settle, the water being decanted to lower the concentration of adhesive agents. This operation is repeated several times with about 300% by volume of deionized water to obtain water-containing beads substantially free of agglomerating adhesive agents. These water-wet beads are dried by spreading them on a tray and passing warm air at a temperature of about 110°F. thereover. The water on and within the beads simply evaporates, and the drying process is quite effective, enabling one to obtain beads which are sufficiently dry to be combined with

-11-

isocyanate-functional liquids without inducing reaction with water.

Example 2

To a stainless steel kettle equipped with an agitator, add 19.12 pounds of mineral oil and then slowly add 24.96 pounds of the dried beads obtained in Example 1 and mix until a uniform dry crumbly paste is formed. This paste has a "silky" feel and can be rubbed onto the skin to provide an emollient effect.

Example 3

To a stainless steel kettle equipped with an agitator, add 24.13 pounds of volatile silicone (Union Carbide Corp. product Silicone #7158 may be used) and then slowly add 17.31 pounds of the dried beads obtained in Example 1 and mix until a uniform dry crumbly paste is formed. This paste has a "silky" feel and is very smooth. It can be rubbed onto the skin to provide a water repellent emollient effect.

Further illustrations of this invention are tabulated below.

TABLE I

| Formula No. | 1 | 2 | 3 | 4 | 5 |
|---|---|---|---|---|---|
| Ingredient (% by Wt.) | | | | | |
| White Pigmented Beads | 50.00 | 25.00 | 16.70 | 15.80 | 47.5 |
| Silicone #7158 (UCC) | | | | | 47.5 |
| Fumed Silica | | | | 5.00 | 5.0 |
| Isopropyl Myristate | 50.00 | 25.00 | 16.70 | 15.80 | |
| Talc | | 50.00 | 66.60 | 63.40 | |
| Total. | 100.00 | 100.00 | 100.00 | 100.00 | 100.0 |
| Appearance | Slightly Lumpy | Lumpy | V. Sl. Lumpy | Fine Powder | Sl. Lumpy |

-12-
TABLE II

| Formula No. | 6 | 7 | 8 |
|---|---|---|---|
| Ingredient (% by Wt.) | | | |
| White Pigmented Beads | 80.00 | 47.50 | 49.50 |
| Silicone SF96-200 | | 47.50 | |
| Mineral Oil | | | 49.50 |
| Fumed Silica | | 5.00 | 1.00 |
| Isopropyl Myristate | 20.00 | | |
| Total. | 100.00 | 100.00 | 100.00 |
| Appearance | Lumpy | Fine Powder | Lumpy |

CLAIMS

1.    Vesiculated beads of cross-linked resin, said beads containing an average of at least 2 thin-walled foraminous cells to provide communication between the vesicles within said bead and the exterior of said beads to allow liquid to move into and out of said beads, said beads being substantially dry and containing an oily liquid.

2.    Vesiculated beads as recited in claim 1 in which at least some of said cell walls are discontinuous as determined by electron microscopy, and there are an average of at least 5 thin-walled cells per bead.

3.    Vesiculated beads as recited in claim 1 in which said beads are constituted by a carboxyl-functional unsaturated polyester resin cross-linked with an ethylenically unsaturated monomer copolymerizable therewith and have a ratio of granular diameter to mean vesicle diameter of at least 5:1, a vesicle volume of from 5% to 95% of the volume of the granule, and not more than about 60% pigment, by volume.

4.    Vesiculated beads as recited in claim 3 in which said polyester resin contains maleate unsaturation and is cross-linked with styrene or vinyl toluene.

5.    Vesiculated beads as recited in claim 1 in which said oily liquid comprises a long chain ester.

6.    Vesiculated beads as recited in claim 1 in which said beads contain mineral oil.

7.    Vesiculated beads as recited in claim 1 in which said beads contain a methyl-terminated polysiloxane oil.

8.    Vesiculated beads as recited in claim 1 in which said beads contain from about 3% up to about 65% of said oily liquid based on the weight of the oil-containing beads and further including talc or clay.

9. A dry powder composition as recited in claim 8 in the form of a pressed cake.

10. A process of incorporating an oily liquid into hollow plastic beads in a manner permitting said oily liquid to leave said beads when said beads are spread on a surface comprising, mixing substantially dry vesiculated beads of cross-linked resin containing an average of at least 2 thin-walled foraminous cells with said oily liquid, and holding said beads in admixture with said oily liquid to allow said liquid to enter the cells of said beads and form a powder therewith.